# EUROPEAN PATENT APPLICATION

(11) **EP 3 900 793 A1**
(43) Date of publication of application: **27.10.2021**
(21) Application number: 20170329.5
(22) Date of filing: 20.04.2020
(51) Int. Cl.: A61Q 19/00, A61K 8/02, A61K 8/04, A61K 8/73, A61K 8/81, A61K 8/87, A61K 8/41

(54) **PEEL-OFF MASK COMPOSITION**

(71) Applicant: Covestro Deutschland AG, 51373 Leverkusen (DE)
(72) Inventor: The designation of the inventor has not yet been filed
(74) Representative: Levpat

(57) **Abstract**

The invention relates to a peel-off mask composition, a method for preparing the same and a method for applying and using the same. Said mask composition comprises the following components: a) at least an aqueous polyurethane dispersion comprising a polyurethane and water; b) at least an organic semi-synthetic water-soluble thickener; c) at least one of acrylic homopolymers and crosslinked copolymers; d) at least a neutralizer; and e) optionally an additive; wherein the amount of the polyurethane is 4 wt% to 24 wt%, the amount of the organic semi-synthetic water-soluble thickener is 0.1 wt% to 2 wt%, the sum of the amounts of the acrylic homopolymers and crosslinked copolymers is 0.1 wt% to 1 wt%, all based on the amount of the mask composition being 100 wt%; the crosslinked copolymer is a product obtained by the crosslinking reaction of a system comprising at least one of acrylate compounds and acrylic acid compounds, at least one of sucrose allyl ether and pentaerythritol allyl ether, and an alkanol acrylate having 10 to 30 carbon atoms.

## Description

### Technical field

The invention relates to a peel-off mask composition, a method for preparing the same and a method for applying and using the same.

### Prior art

The peel-off or peelable masks that currently exist on the market are those used as follows: applying a liquid in the form of an emulsion or a paste or a spray uniformly to the face, waiting for about 15 to 20 minutes to dry and form a film, and peeling it off. The peel-off or peelable masks are required to be able to form a continuous film in a short time and not to break during the peeling process. Thus, the film has to have good elasticity and tensile strength, and a moderate adhesion between the film and the skin.

When the masks comprise polyvinyl alcohols or polyvinyl pyrrolidones as a film-forming agent, it is very painful on the skin during the peeling process of the film due to the strong adhesion between the film and the skin. When the masks comprise an acrylic film-forming agent, the film cannot be completely removed from the skin regardless of whether it is wetted with water or not, since the film with the film-forming agent is too hard and easily breaks. When the masks comprise polyurethanes as a film-forming agent, the film can be completely removed from the skin after being wetted. However, this is only fulfilled when the mask is applied to the skin in such a large amount that it covers the color of the skin itself, or when more polyurethane is added to the masks as a film-forming agent.

TW201000148 discloses a cosmetic composition for applying on the skin comprising one or more polyurethane, said polyurethane containing at least one sequence selected from the group consisting of polyether sequences, polycarbonate sequences and polyether-polycarbonate sequences.

CN104352359 discloses a peel-off mask substrate based on an aqueous polyurethane dispersion, which comprises the aqueous polyurethane dispersion as a main ingredient, and additives such as thickener, emulsifier, fat or oil, preservative, essence and the like. The mask substrate may specifically comprise an aqueous polyurethane dispersion, hydroxyethyl cellulose, and Carbopol.

CN104546549 discloses a mask composition containing a sunscreen filter agent which is peelable from the skin, comprising a polyurethane, a thickener, a water-insoluble sunscreen filter and water. The weight ratio of the polyurethane and the oil phase in the mask composition is not less than 2: 3. The mask composition may specifically comprise one of Carbopol Ultrez 21 and hydroxyethyl cellulose, and an aqueous polyurethane dispersion.

It is desirable to develop a mask composition which is able to form a film with good elasticity and peel-off integrity on the skin surface when used in a small amount, and shows a short drying time.

### Detailed description of the invention

Objects of the present invention are to provide a peel-off mask composition, a method for preparing the same and a method for applying and using the same.

A peel-off mask composition according to the present invention comprises the following components:
a) at least an aqueous polyurethane dispersion comprising a polyurethane and water;
b) at least an organic semi-synthetic water-soluble thickener;
c) at least one of acrylic homopolymers and crosslinked copolymers;
d) at least a neutralizer; and
e) optionally an additive;
wherein the amount of the polyurethane is 4 wt% to 24 wt%, the amount of the organic semi-synthetic water-soluble thickener is 0.1 wt% to 2 wt%, the sum of the amounts of the acrylic homopolymers and crosslinked copolymers is 0.1 wt% to 1 wt%, all based on the amount of the mask composition being 100 wt%;
the crosslinked copolymer is a product obtained by the crosslinking reaction of a system comprising at least one of acrylate compounds and acrylic acid compounds, at least one of sucrose allyl ether and pentaerythritol allyl ether, and an alkanol acrylate having 10 to 30 carbon atoms;
the viscosity of the acrylic homopolymer is configured as follows:
the viscosity of the aqueous acrylic homopolymer solution is less than 10000 mPa·s when
the concentration of the aqueous solution is 0.2%, and
the viscosity of the aqueous acrylic homopolymer solution is less than 15000 mPa·s when
the concentration of the aqueous solution is 0.5%;
the viscosity of the crosslinked copolymer is configured as follows:
the viscosity of the aqueous crosslinked copolymer solution is less than 5000 mPa·s when
the concentration of the aqueous solution is 0.2%, and
the viscosity of the aqueous crosslinked copolymer solution is less than 8000 mPa·s when
the concentration of the aqueous solution is 0.5%;
the viscosity being measured with the aqueous solutions adjusted to a pH value of 7.3 to 7.8
at 20 rpm using a Brookfield viscometer at 25 °C;
the aqueous solution being prepared by dispersing the acrylic homopolymers or crosslinked copolymers in water homogeneously. The above weight values are all weight values of the effective or solid components of the composition.

According to one aspect of the present invention, a method for preparing a peel-off mask composition according to the present invention is provided, including the following steps:
i) mixing component b) organic semi-synthetic water-soluble thickener and component c) at least one of acrylic homopolymers and crosslinked copolymers to obtain a mixture; and
ii) mixing the mixture and component a) aqueous polyurethane dispersion to obtain said mask composition;
component d) and optionally component e) being each independently added in step i) or step ii).

According to another aspect of the present invention, use of the peel-off mask composition according to the present invention for applying on the skin to take care of the skin is provided.

According to yet another aspect of the present invention, a method for taking care of the skin is provided, including:
applying the peel-off mask composition according to the present invention on the skin surface and drying to form a film;
wetting the film and peeling it off from the skin.

The peel-off mask composition of the present invention comprises component b) organic semi-synthetic water-soluble thickener, and component c) at least one of acrylic homopolymers and crosslinked copolymers. The mask composition is able to form a tough, continuous, elastic and stretchable film on the skin surface, is not easy to break and is present as an integral film when being peeled off from the skin. In addition, the mask composition of the present invention can dry quickly to form a film after being applied to the skin, which greatly reduces the time for the user to wait for the mask to dry. The mask composition of the present invention only needs to be applied in a small amount on the skin surface and can form a film with good elasticity and peel-off integrity. The mask composition of the present invention is convenient to use and has good user experience.

### Embodiments

The present invention provides a peel-off mask composition, comprising the following components: a) at least an aqueous polyurethane dispersion comprising a polyurethane and water; b) at least an organic semi-synthetic water-soluble thickener; c) at least one of acrylic homopolymers and crosslinked copolymers; d) at least a neutralizer; and e) optionally an additive; wherein the amount of the polyurethane is 4 wt% to 24 wt%, the amount of the organic semi-synthetic water-soluble thickener is 0.1 wt% to 2 wt%, the sum of the amounts of the acrylic homopolymers and crosslinked copolymers is 0.1 wt% to 1 wt%, all based on the amount of the mask composition being 100 wt%;
the crosslinked copolymer is a product obtained by the crosslinking reaction of a system comprising at least one of acrylate compounds and acrylic acid compounds, at least one of sucrose allyl ether and pentaerythritol allyl ether, and an alkanol acrylate having 10 to 30 carbon atoms;
the viscosity of the acrylic homopolymer is configured as follows:
the viscosity of the aqueous acrylic homopolymer solution is less than 10000 mPa·s when
the concentration of the aqueous solution is 0.2%, and
the viscosity of the aqueous acrylic homopolymer solution is less than 15000 mPa·s when
the concentration of the aqueous solution is 0.5%;
the viscosity of the crosslinked copolymer is configured as follows:
the viscosity of the aqueous crosslinked copolymer solution is less than 5000 mPa·s when
the concentration of the aqueous solution is 0.2%, and
the viscosity of the aqueous crosslinked copolymer solution is less than 8000 mPa·s when
the concentration of the aqueous solution is 0.5%;
the viscosity being measured with the aqueous solutions adjusted to a pH value of 7.3 to 7.8 at 20 rpm using a Brookfield viscometer at 25 °C;
the aqueous solutions being prepared by dispersing the acrylic homopolymers or crosslinked copolymers in water homogeneously.

The invention also provides a method for preparing the mask composition and a method for applying and using the same.

### Component a) aqueous polyurethane dispersion

Within the scope of the present invention, the term "water-insoluble and water-nondispersible isocyanate-functionalized polyurethane prepolymer" means that the prepolymer has a solubility in water at 23 °C of less than 10 g/liter, preferably less than 5 g/liter, and the prepolymer is unable to produce a sedimentation stable dispersion in water (especially deionized water) at 23 °C. In other words, the prepolymer precipitates out in experiments in which it is dispersed in water.

The aqueous polyurethane dispersion may be added to the mask composition in the form of a solid or a dispersion, and is preferably added to the mask composition in the form of a dispersion, that is, added to the mask composition in the form of an aqueous polyurethane dispersion. When being added to the mask composition in the form of a solid, the polyurethane may be dispersed in water of the mask composition to form an aqueous polyurethane dispersion.

For a composition in the form of a dispersion, the weight of the polyurethane (i.e. the weight of the effective components of the aqueous polyurethane dispersion or the weight of the solid components of the aqueous polyurethane dispersion) = the weight of the dispersion × the content of the solid components of the dispersion. For a solid substance containing crystal water, the weight of the polyurethane (i.e. the weight of the effective components or the weight of the solid components) = the weight of the substance - the weight of the crystal water.

The amount of the polyurethane is 8 wt% to 15 wt%, based on the amount of the mask composition being 100 wt%.

The polyurethane is preferably obtained by the reaction of a system comprising a1) at least a water-insoluble and water-nondispersible isocyanate-functionalized polyurethane prepolymer and a2) at least an isocyanate-reactive compound.

The polyurethane comprises preferably at least a sulfonic acid group and/or sulfonate group.

The polyurethane is preferably an anionic polyurethane.

The anionic polyurethane is preferably obtained by the reaction of a system comprising a1) at least a water-insoluble and water-nondispersible isocyanate-functionalized polyurethane prepolymer and a2) at least an isocyanate-reactive compound.

The anionic polyurethane comprises preferably at least a sulfonic acid group and/or sulfonate group, and most preferably at least a sodium sulfonate group.

The anionic polyurethane may be linear or branched, and is most preferably linear.

The number average molecular weight of the anionic polyurethane is preferably 100,000 g/mol to 500,000 g/mol, further preferably 120,000 g/mol to 400,000 g/mol, and most preferably 150,000 g/mol to 400,000 g/mol.

### a1) Water-insoluble and water-nondispersible isocyanate-functionalized polyurethane prepolymer

The water-insoluble and water-nondispersible isocyanate-functionalized polyurethane prepolymer has preferably a terminal isocyanate group, that is, the isocyanate group is located at the end of the prepolymer chain, and most preferably, all the chain ends of the polyurethane prepolymer have isocyanate group.

The water-insoluble and water-nondispersible isocyanate-functionalized polyurethane prepolymer is substantially free of ionic groups and/or ionogenic groups, that is, the content of the ionic groups and/or ionogenic groups is preferably less than 15 mg equivalent/100 g of polyurethane prepolymer, further preferably less than 5 mg equivalent/100 g of polyurethane prepolymer, more preferably less than 1 mg equivalent/100 g of polyurethane prepolymer, most preferably less 0.1 mg equivalent/100 g of polyurethane prepolymer. The water here means deionized water without any surfactant added.

The water-insoluble and water-nondispersible isocyanate-functionalized polyurethane prepolymer can be prepared by the reaction of a system comprising a polyol, an organic polyisocyanate, optionally a hydroxyl-functional compound having a number average molecular weight of 62 to 399 g/mol, and optionally a non-ionic hydrophilizing reagent.

The polyol is preferably one or more selected from polyether polyols, polycarbonate polyols, polyether polycarbonate polyols, and polyester polyols, further preferably a polyol having a linear structure, and still more preferably one or more selected from polytetramethylene glycol polyethers and polycarbonate polyols, most preferably polytetramethylene glycol polyethers.

The number average molecular weight of the polyol is preferably 400 g/mol to 6000 g/mol, and most preferably 600 g/mol to 3000 g/mol.

The hydroxyl functionality of the polyol is preferably 1.5 to 6, more preferably 1.8 to 3, and most preferably 1.9 to 2.1.

The isocyanate group (NCO) functionality of the organic polyisocyanate is preferably not less than 2.

The organic polyisocyanate is preferably one or more selected from aliphatic polyisocyanate, aromatic polyisocyanate, and alicyclic polyisocyanate, further preferably one or more selected from 1,4-butylene diisocyanate, 1,6-hexamethylene diisocyanate (HDI), isophorone diisocyanate (IPDI), 2,2,4- and/or 2,4,4-trimethylhexamethylene diisocyanate, bis(4,4'-isocyanatocyclohexyl) methane isomers or mixtures of these isomers, 1,4-cyclohexylene diisocyanate, 4-isocyanatomethyl-1,8-octane diisocyanate (nonane triisocyanate), 1,4-benzene diisocyanate, 2,4- and/or 2,6-toluene diisocyanate, 1,5-naphthylene diisocyanate, 2,2'- and/or 2,4'- and/or 4,4'-diphenylmethane diisocyanate, 1,3- and/or 1,4-bis(2-isocyanatoprop-2-yl) benzene (TMXDI), 1,3-bis(isocyanatomethyl)benzene (XDI) and C1-C8-alkyl 2,6-diisocyanatohexanoate (lysine diisocyanate), most preferably one or more selected from hexamethylene diisocyanate and isophorone diisocyanate.

The organic polyisocyanate may also be a polyisocyanate containing a hetero atom in the groups bonded to isocyanate groups, for example, a polyisocyanate obtained by modification using uretdione, isocyanurate, urethane, allophanate, biuret, carbodiimide, iminooxadiazinedione and/or oxadiazinetrione structure.

The hydroxy-functional compound having a molecular weight of 62 to 399 mol/g is preferably one or more selected from non-polymeric polyols having no more than 20 carbon atoms, ester diols, and monofunctional hydroxy compounds.

The non-polymeric polyol having no more than 20 carbon atoms is preferably one or more selected from ethylene glycol, diethylene glycol, triethylene glycol, 1,2-propanediol, 1,3-propanediol, 1,4-butanediol, 1,3-butanediol, cyclohexanediol, 1,4-cyclohexane dimethanol, 1,6-hexanediol, neopentyl glycol, hydroquinone dihydroxyethyl ether , bisphenol A (2,2-bis(4-hydroxyphenyl) propane), hydrogenated bisphenol A (2,2-bis(4-hydroxycyclohexyl) propane), trimethylol propane, trimethylol ethane, glycerol and pentaerythritol.

The ester diol is preferably one or more selected from α-hydroxybutyl ε-hydroxyhexanoate, ω-hydroxyhexyl γ-hydroxybutyrate, (β-hydroxyethyl) adipate and di(β-hydroxyethyl) terephthalate.

The monofunctional hydroxy compound is preferably one or more selected from ethanol, n-butanol, ethylene glycol monobutyl ether, diethylene glycol monomethyl ether, diethylene glycol monobutyl ether, propylene glycol monomethyl ether, dipropylene glycol monomethyl ether, tripropylene glycol monomethyl ether, dipropylene glycol monopropyl ether, propylene glycol monobutyl ether, dipropylene glycol monobutyl ether, tripropylene glycol monobutyl ether, 2-ethyl hexanol, 1-octanol , 1-dodecanol, and 1-hexadecanol.

The non-ionic hydrophilicizing agent is preferably one or more selected from polyoxyalkylene ethers containing a hydroxyl group, an amino group and a thiol group.

The non-ionic hydrophilizing agent is preferably a monohydroxy-functionalized polyoxyalkylene polyether having 5 to 70, preferably 7 to 55 ethylene oxide units per molecule. The polyoxyalkylene polyether can be obtained by alkoxylation of a suitable starter molecule according to known methods.

The monohydroxy-functionalized polyoxyalkylene polyether is preferably pure polyoxyethylene polyether or mixed polyoxyalkylene polyethers. The polyoxyethylene polyether contains preferably not less than 30 mol%, and most preferably not less than 40 mol% of ethylene oxide units, based on the alkylene oxide units being 100 mol%.

### a2) Isocyanate-reactive compound

The isocyanate-reactive compound is preferably one or more selected from amino-functional compounds and hydroxy-functional compounds.

The amino-functional compound is preferably one or more selected from primary amines, secondary amines and diamines, most preferably diamines.

The amino-functional compound is preferably an amino-functional compound without any ionic group or ionogenic group, or an amino-functional compound with an ionic group or ionogenic group.

The amino-functional compound without any ionic group or ionogenic group is preferably one or more selected from organic diamines, polyamines, compounds containing a primary and secondary amino group, compounds containing a (primary or secondary) amino group and a hydroxyl groups, and monofunctional isocyanate-reactive amine compounds.

The organic diamine is preferably one or more selected from 1,2-ethylene diamine, 1,2- and 1,3-diaminopropane, 1,4-diaminobutane, 1,6-diaminohexane, isophorone diamine, isomer mixtures of 2,2,4- and 2,4,4-trimethylhexamethylene diamine, 2-methylpentamethylene diamine, 4,4-diaminodicyclohexylmethane, hydrazine hydrate and dimethylethylene diamine.

The polyamine is preferably diethylene triamine.

The compound containing a primary and secondary amino group is preferably one or more selected from diethanol amine, 3-amino-1-methylaminopropane, 3-amino-1-ethylaminopropane, 3-amino-1-cyclohexylaminopropane and 3-amino-1-methylaminobutane.

The compound containing a (primary or secondary) amino group and a hydroxyl groups, the alcohol amine is preferably one or more selected from N-aminoethyl ethanolamine, ethanolamine, 3-aminopropanol, and neopentanol amine.

The monofunctional isocyanate-reactive amine compound is preferably one or more selected from methylamine, ethylamine, propylamine, butylamine, octylamine, laurylamine, stearylamine, isononyloxypropylamine, dimethylamine, diethylamine, dipropylamine, dibutylamine, N-methylaminopropylamine, diethyl(methyl)aminopropylamine, morpholine, piperidine and suitable substituted derivatives thereof.

The amino-functional compound without any ionic group or ionogenic group is most preferably a diamine without any ionic group or ionogenic group.

The amino-functional compound with an ionic group or ionogenic group is preferably an anionic hydrophilized compound.

The anionic hydrophilized compound is preferably one or more selected from 2-(2-aminoethylamino)ethanesulfonic acid, ethylenediamine-propyl or butyl-sulfonic acid, 1,2- or 1,3-propanediamine-β-ethylsulfonic acid and taurine and salts thereof, further preferably those containing a sulfonate group as an ionic group and two amino groups, most preferably one or more of the following: 2-(2-aminoethylamino)ethylsulfonate and 1,3-propanediamine-β-ethylsulfonate.

The hydroxy-functional compound is preferably a polymer polyol, and further preferably one or more selected from polyester polyols, polyacrylate polyols, polyurethane polyols, polycarbonate polyols, polyether polyols, polyester polyacrylate polyols, polyurethane polyacrylate polyols, polyurethane polyester polyols, polyurethane polyether polyols, polyurethane polycarbonate polyols, and polyester polycarbonate polyols, and more preferably one or more selected from polytetramethylene glycol polyethers and polycarbonate polyols, most preferably polytetramethylene glycol polyethers.

The aqueous anionic polyurethane dispersion is preferably one or more selected from polyurethane-34, polyurethane-32, polyurethane-35, polyurethane-48, polyurethane-11 and polyurethane-2, the above names all being according to the International Cosmetic Ingredient Dictionary and Handbook (INCI); most preferably one or more selected from Baycusan®C1000/1, Baycusan®C1001/1, Baycusan®C1003/1, Baycusan®C1004/1, Baycusan®C1008/1, Baycusan®C1010, Carfil®9221, Carfil® UV35, Carfil® 9235NP and Carfil® ST11.

### Preparation of aqueous anionic polyurethane dispersions

The method for preparing aqueous anionic polyurethane dispersions includes the following steps:
preparing a1) a water-insoluble and water-nondispersible isocyanate-functionalized polyurethane prepolymer;
reacting a1) the polyurethane prepolymer with a2) an isocyanate-reactive compound; and dispersing the anionic polyurethane in water before, during or after the reaction.

The anionic groups contained in the aqueous anionic polyurethane dispersion are introduced by the amino-functional compounds with an ionic group or ionogenic group as the isocyanate-reactive compounds.

The preparation of aqueous anionic polyurethane dispersions can be performed in a homogeneous phase in one or more stages, or partly in a dispersed phase for a multi-stage reaction. The method for preparing the aqueous anionic polyurethane dispersions may be the prepolymer mixing method, the acetone method, or the melt dispersing method, and is preferably the acetone method.

The equivalent ratio of the isocyanate groups to the isocyanate-reactive groups is preferably 1.05 to 3.5, more preferably 1.1 to 3.0, and most preferably 1.1 to 2.5.

The particle size of the anionic polyurethanes is preferably less than 750 nm, and most preferably less than 500 nm. The particle size of the present invention is measured using a Malvern Zetasizer 1000 from Malver after diluting the anionic polyurethane with deionized water.

The solid content of the aqueous anionic polyurethane dispersion is preferably 5 wt% to 50 wt%, and most preferably 30 wt% to 45 wt%, based on the amount of the aqueous anionic polyurethane dispersion being 100 wt%. The solid content is calculated after heating the weighed sample to a constant weight at 125 °C and reweighing the sample under the constant weight.

### Component b) organic semi-synthetic water-soluble thickener

The organic semi-synthetic water-soluble thickener of the present invention is a substance capable of changing the rheological properties of the solution which is obtained by chemical modification of a natural substance.

The cellulose of the present invention refers to a linear polymer obtained by bonding a plurality of β-D-glucose molecules with 1,4-glycosidic bonds.

The modified cellulose of the present invention is chemically modified cellulose having water solubility, and is a water-soluble polymer composed of a mixture of two polysaccharide substances such as amylose and highly branched amylopectin.

The amount of component b) organic semi-synthetic water-soluble thickener is preferably 0.2 wt% to 1 wt%, and most preferably 0.3 wt% to 0.5 wt%, based on the amount of the mask composition being 100 wt%. When the organic semi-synthetic water-soluble thickener is in the form of a dispersion or a substance containing crystal water, the amount of the organic semi-synthetic water-soluble thickener refers to the amount of the effective or solid component.

Component b) organic semi-synthetic water-soluble thickener is preferably one or more selected from modified celluloses and modified starches.

The modified cellulose has a β-D-glucose ring with three hydroxyl groups which can undergo the nucleophilic substitution reaction, one-to-one reaction, or branching. The substituent is preferably one or more selected from methyl, ethyl, carboxymethyl, and hydroxyalkyl.

The modified cellulose has a β-D-glucose ring with three hydroxyl groups which can undergo the nucleophilic substitution reaction, and is more preferably one or more selected from cellulose ethers and derivatives thereof, most preferably one or more selected from methyl cellulose, hydroxypropyl methyl cellulose, carboxymethyl cellulose, hydroxyethyl cellulose, and hydroxypropyl cellulose.

The modified starch is preferably one or more selected from modified amyloses and modified amylopectins, most preferably aluminum starch octenylsuccinate.

Component b) organic semi-synthetic water-soluble thickener is more preferably a polyhydric natural gum derivative, and most preferably hydroxyethyl cellulose.

### Component c) acrylic homopolymers and crosslinked copolymers

The homopolymer of the present invention refers to a polymer obtained by polymerizing one monomer.

The crosslinked copolymer of the present invention is a polymer with a relatively high molecular mass composed of a large hydrophilic portion and a small lipophilic portion. The lipophilic portion is adsorbed at the oil-water interface, and the hydrophilic portion swells in the aqueous phase, which makes up a stable emulsion. The crosslinked copolymer of the present invention can thicken the water phase and can also improve the stability of the emulsion.

Component c) is one or more of acrylic homopolymers and crosslinked copolymers.

The acrylic homopolymer has preferably the following structure:

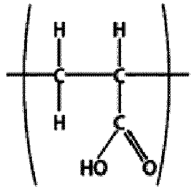

The acrylic homopolymer is more preferably a homopolymer of a system comprising A) acrylic acid and B) at least one of pentaerythritol allyl ether, sucrose allyl ether and propenyl allyl ether, most preferably one or more selected from Carbopol® 941, Carbopol® 981, Carbopol® ETD 2050, Ashland™ 981-Carbomer, Synthalen®, Polygel® CB, TC-CARBOMER 381, TC-CARBOMER 341, AQUPEC® 801EG-300, AQUPEC® HV-501E, TEGO® Carbomer 141 and OptaSense G41.

Preferably, the viscosity of the acrylic homopolymer is configured as follows: the viscosity of the aqueous acrylic homopolymer solution is less than 10000 mPa·s when the concentration of the aqueous solution is 0.2%, and the viscosity of the aqueous acrylic homopolymer solution is less than 15000 mPa·s when the concentration of the aqueous solution is 0.5%. The viscosity is measured with the aqueous solutions adjusted to a pH value of 7.3 to 7.8 at 20 rpm using a Brookfield viscometer at 25 °C. The aqueous solutions are prepared by dispersing the acrylic homopolymers in water homogeneously.

Most preferably, the viscosity of the acrylic homopolymer is configured as follows: the viscosity of the aqueous acrylic homopolymer solution is more than or equal to 500 mPa·s and less than 10000 mPa·s when the concentration of the aqueous solution is 0.2%, and the viscosity of the aqueous acrylic homopolymer solution is more than or equal to 500 mPa·s and less than 15000 mPa·s when the concentration of the aqueous solution is 0.5%.

The pH value of the aqueous solutions can be adjusted using a neutralizer, which may be the same as or different from component d) according to the present invention.

The crosslinked copolymer is a product obtained by the crosslinking reaction of a system comprising I) at least one of acrylate compounds and acrylic acid compounds, II) at least one of sucrose allyl ether and pentaerythritol allyl ether, and III) an alkanol acrylate having 10 to 30 carbon atoms.

The crosslinked copolymer has more preferably the following structure:

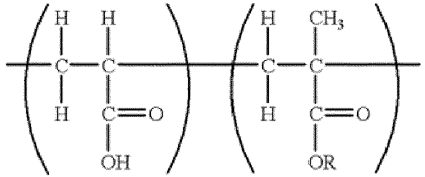

The crosslinked copolymer comprises preferably a hydrophobic group.

The crosslinked copolymer is most preferably one or more selected from Pemulen™ TR-2, Carbopol® SC-500 Polymer, AQUPEC® SER W-150C and AQUPEC® SER W-300C.

Preferably, the viscosity of the crosslinked copolymer is configured as follows: the viscosity of the aqueous crosslinked copolymer solution is less than 5000 mPa·s when the concentration of the aqueous solution is 0.2%, and the viscosity of the aqueous crosslinked copolymer solution is less than 8000 mPa·s when the concentration of the aqueous solution is 0.5%. The viscosity is measured with the aqueous solutions adjusted to a pH value of 7.3 to 7.8 at 20 rpm using a Brookfield viscometer at 25 °C. The aqueous solutions are prepared by dispersing the crosslinked copolymers in water homogeneously.

Most preferably, the viscosity of the crosslinked copolymer is configured as follows: the viscosity of the aqueous crosslinked copolymer solution is more than or equal to 500 mPa·s and less than 5000 mPa·s when the concentration of the aqueous solution is 0.2%, and the viscosity of the aqueous crosslinked copolymer solution is more than or equal to 500 mPa·s and less than 8000 mPa·s when the concentration of the aqueous solution is 0.5%.

The pH value of the aqueous solutions can be adjusted using a neutralizer, which may be the same as or different from component d) according to the present invention.

The sum of the amounts of the acrylic homopolymers and crosslinked copolymers is preferably 0.2 wt% to 0.6 wt%, based on the amount of the mask composition being 100 wt%. When the acrylic homopolymer or crosslinked copolymer is in the form of a dispersion or a substance containing crystal water, the amount of the acrylic homopolymer or crosslinked copolymer refers to the amount of the effective or solid component.

### Additional acrylic homopolymers and/or crosslinked copolymers

The mask composition may further comprises an additional acrylic homopolymer and/or crosslinked copolymer, the content of which being less than the amount of component c) added in the composition.

The additional acrylic homopolymer is preferably a carbomer different from the carbomer of component c), and most preferably one or more selected from Carbopol® 940, Carbopol® 980, Carbopol® Ultrez 10, Carbopol® 934, Carbopol® Ultrez 30, Carbopol® 5984 Polymer, Carbopol® 2984 Polymer, Carbopol® Clear Polymer, Carbopol® Style 2.0, Carbopol® Silk 100 Polymer, Ashland™ 940 Carbomer, Ashland™ 980 Carbomer, TC-CARBOMER 380, TC-CARBOMER 390, TC-CARBOMER 356, TC-CARBOMER 276, TC-CARBOMER 340, TC-CARBOMER 351, TC-CARBOMER 640, TC-CARBOMER 334, AQUPEC 805EG-300, AQUPEC HV-505E, AQUPEC HV-504E, TEGO® Carbomer 134, TEGO® Carbomer 140, TEGO® Carbomer 340FD, Rheocare® C Plus, OptaSense™ G40, SuperGel™ CE, SuperGel™ SK and Polygel CA.

The additional crosslinked copolymer is preferably one or more selected from Carbopol® Ultrez 20, Carbopol® Ultrez 21, Carbopol® ETD2020, Pemulen TR-1, TC-CARBOMER FD2010, TC-CARBOMER FD21 and TEGO® Carbomer 341ER.

### Component d) Neutralizer

The neutralizer can adjust the pH value of component c) to 4 to 8.5, most preferably 6.5 to 7.0.

The neutralizer is preferably one or more selected from sodium hydroxide, triethanol amine, aminomethyl propanol, ammonium hydroxide, potassium hydroxide, arginine, tetrahydroxypropyl ethylenediamine, tromethamine, diisopropanol amine and triisopropanol amine, most preferred triethanol amine.

### Component e) additives

The additives of the present invention are those that are physically and chemically compatible with components in the mask composition of the present invention.

The additive is preferably one or more selected from thickeners, emulsifiers, co-emulsifiers, fats or oils, preservatives, humectants, dyes, non-volatile solvents, diluents, pearlescing additives, foam promoters, additive surfactants, nonionic co-surfactants, pH adjusters, perfumes, chelating agents, proteins, skin activators, sunscreens, UV absorbers and vitamins.

The thickener is different from component c) and is preferably one or more selected from diatomaceous earth, Carbopol, starch, gelatin, sodium alginate, casein, guar gum, chitosan, gum arabic, xanthan gum, natural lanolin and agar.

The amount of the thickener may be an amount well known to those skilled in the art, and the amount of the thickener is preferably 0.1 to 10 wt%, based on the amount of the mask composition being 100 wt%.

The emulsifier is preferably one or more selected from carboxylates, sulfates, sulfonates, amine derivatives, alkyl ethers, stearyl esters, polyoxyethylene polyethers, and polyoxypropylene polyethers.

The amount of the emulsifier may be an amount well known to those skilled in the art, and the amount of the emulsifier is preferably 0.1 to 10 wt%, based on the amount of the mask composition being 100 wt%.

The fat or oil is preferably one or more selected from vegetable oils, animal fats, mineral oils, and synthetic fats or oils, and most preferably one or more selected from olive oil, coconut oil, castor oil, cottonseed oil, soybean oil , sesame oil, almond oil, peanut oil, corn oil, rice bran oil, tea seed oil, sea buckthorn oil, avocado oil, candlenut oil, european nut oil, walnut oil, cocoa oil, mink oil, yolk oil, lanolin oil, lecithin, squalane, lanolin derivatives, polysiloxanes, fatty acids, fatty alcohols, fatty acid esters, and vaseline.

The amount of the fat or oil may be an amount well known to those skilled in the art, and the amount of the fat or oil is preferably 0.1 to 30 wt%, based on the amount of the mask composition being 100 wt%.

The preservative is preferably one or more selected from alcohol preservatives, donors of formaldehyde and preservatives of aldehyde derivatives, preservatives of benzoic acid and derivatives thereof, sodium hydroxymethylglycine, p-hydroxy acetophenone, and preservative compounds, most preferably one or more selected from compounds of phenoxyethanol and ethylhexyl glycerol, and compounds of methyl propylene glycol, caprylyl glycol, and phenylpropanol. The compound of methyl propylene glycol, caprylyl glycol and phenylpropanol is preferably Dermosoft OMP.

The amount of the preservative may be an amount well known to those skilled in the art, and the amount of the preservative is preferably 0.3 to 2 wt%, based on the amount of the mask composition being 100 wt%.

The humectant is preferably one or more selected from polyols, amides, lactic acids and sodium lactates, sodium pyrrolidonecarboxylate, glucose esters, collagens, chitin derivatives, and chitosans, and most preferably polyols.

The amount of the humectant may be an amount well known to those skilled in the art, and the amount of the humectant is preferably 0.1 to 20 wt%, based on the amount of the mask composition being 100 wt%.

### Cosmetically acceptable medium

The mask composition may further comprises a cosmetically acceptable medium. The cosmetically acceptable medium can be selected based on other ingredients in the mask composition.

The cosmetically acceptable medium is preferably one or more selected from water and aqueous solutions of lower alkanols, more preferably water, and most preferably deionized water.

The aqueous solution of a lower alkanol comprises preferably a monohydric alcohol having 1 to 6 carbon atoms, and most preferably one or more selected from ethanol and isopropanol.

The amount of the cosmetically acceptable medium may be an amount well known to those skilled in the art and the amount of the cosmetically acceptable medium is preferably 20 to 99 wt%, more preferably 30 to 99 wt%, and most preferably 40 to 80 wt%, based on the amount of the mask composition being 100 wt%

### Method for preparing a mask composition

Component d) neutralizer, optionally component e) additive and cosmetically acceptable medium can be each independently added in step i) or step ii).

Preferably, the method for preparing the mask composition of the present invention includes the following steps:
dispersing component b) and component c) in water homogeneously to obtain a mixture, and
raising the temperature of the mixture to 70 °C-90 °C;
adding component d) neutralizer and optionally component e) additive to the mixture and stirring well, reducing the temperature to 40 °C-50 °C, and adding component d) neutralizer to adjust the pH value to 4 to 8.5, most preferably 6.5 to 7.0 depending on the situation; and adding component a), stirring well, and adding component d) neutralizer to adjust the pH value to 4 to 8.5, most preferably 6.5 to 7.0, to obtain the mask composition.

### Mask composition

The mask composition may be spray-like, gel-like, cream-like or emulsion-like.

### Examples

Unless defined otherwise, all technical and scientific terms used herein have the same meaning as commonly understood by one of ordinary skill in the art. When the definitions of the terms in this specification conflict with the meanings commonly understood by those skilled in the art, the definitions described herein shall apply.

Unless otherwise stated, all numerical values of the amounts of components, reaction conditions, and the like used in the specification and claims are understood to be modified by the term "about". Therefore, unless indicated to the contrary, the numerical values set forth herein are approximations that can vary depending on the required performance required to be obtained.

As used herein, "and/or" means one or all of the elements mentioned.

As used herein, "including" and "comprising" encompass situations where there are only the mentioned elements, as well as situations where there are other elements in addition to the mentioned elements.

All percentages in the present invention are weight percentages, and are based on the amount of the cosmetic composition of the present invention being 100 wt%, unless otherwise stated.

The molecular weight of the present invention is a number average molecular weight, unless otherwise stated. The number average molecular weight was determined by gel permeation chromatography (GPC) method according to GB/T 21863-2008 "Gel permeation chromatography (GPC) - Tetrahydrofuran as elution solvent".

The analytical measurements of the present invention were all performed at 23 ± 2 °C, unless otherwise stated.

The solid content of the dispersions was measured using a HS153 moisture meter from Mettler Toledo according to DIN-EN ISO 3251.

The pH value was measured using PB-10 pH meter from Sartorius at 23 °C according to DIN 19263.

### Raw materials and reagents

Baycusan® C1004/1: aqueous anionic polyurethane dispersion with a solid content of 41 wt% and INCI name of polyurethane-35, available from Covestro Polymers (China) Co., Ltd.

Baycusan® C1000/1: aqueous anionic polyurethane dispersion with a solid content of 40 wt% and INCI name of polyurethane-34, available from Covestro Polymers (China) Co., Ltd.

Baycusan® C1001/1: aqueous anionic polyurethane dispersion with a solid content of 32 wt% and INCI name of polyurethane-34, available from Covestro Polymers (China) Co., Ltd.

Baycusan® C1008/1: aqueous anionic polyurethane dispersion with a solid content of 30 wt% and INCI name of polyurethane-48, available from Covestro Polymers (China) Co., Ltd.

Carfil 9221: aqueous polyurethane dispersion with a solid content of 40 wt% and INCI name of polyurethane-35, available from Wanhua Chemical Group Co., Ltd.

Carfil ST11: aqueous polyurethane dispersion with a solid content of 45 wt% and INCI names of polyurethane-35 and polyurethane-11, available from Wanhua Chemical Group Co., Ltd.

Euxyl® PE 9010: phenoxyethanol and ethylhexyl glycerol with a content of the effective components of 100 wt%, purchased from Schülke & Mayr GmbH.

Penmulen® TR-2: crosslinked copolymer of an acrylic acid/ester compound and an alkanol acrylate having 10 to 30 carbon atoms with a solid content of 100 wt% and a viscosity of 1700 to 4500 mPa·s (with a concentration of 0.2%) and of 5000 mPa·s (with a concentration of 0.5%), purchased from Lubrizol.

Carbopol® Ultrez 21: crosslinked copolymer of an acrylic acid/ester compound and an alkanol acrylate having 10 to 30 carbon atoms with a solid content of 100 wt% and a viscosity of 25000 mPa·s (with a concentration of 0.2%) and of 52000 mPa·s (with a concentration of 0.5%), purchased from Lubrizol.

Aculyn® 38: crosslinked copolymer of an acrylic acid/ester compound and vinyl neodecanoate with a solid content of 29 wt% and a viscosity of less than 10000 mPa·s (with a concentration of 1%), purchased from The Dow Chemical.

Carbopol® 941: acrylic homopolymer with a solid content of 100% and a viscosity of 1950 to 7000 mPa·s (with a concentration of 0.2%) and of 4000 to 11000 mPa·s (with a concentration of 0.5%), purchased from Lubrizol.

Carbopol® 940: acrylic homopolymer with a solid content of 100% and a viscosity of 25000 mPa·s (with a concentration of 0.2%) and of 40000 to 60000 mPa·s (with a concentration of 0.5%), purchased from Lubrizol.

Carbopol® aqua SF-1: acrylic copolymer with a solid content of 30 wt%, purchased from Lubrizol.

Carbopol® Ultrez 30: acrylic homopolymer with a solid content of 100% and a viscosity of 10000 mPa·s (with a concentration of 0.2%) and of 30000 mPa·s (with a concentration of 0.5%), purchased from Lubrizol.

Natrosol™ 250HHR: hydroxyethyl cellulose with a solid content of 100%, purchased from Ashland.

Stardesign powder: aluminum starch octenylsuccinate with a solid content of 100 wt%, purchased from Cargill.

Triethanol amine: original concentration of 99%, purchased from Shanghai Lingfeng Chemical Reagent Co., Ltd, diluted to 10% in water in the laboratory for future use. PEG-40 hydrogenated castor oil: emulsifier, purchased from Croda.

### Method for preparing mask compositions of Examples and Comparative Examples

The organic semi-synthetic water-soluble thickener, acrylic homopolymer, and crosslinked copolymer were dispersed homogeneously in the aqueous phase according to the composition of the mask compositions of the Examples and Comparative Examples listed in Tables 1 and 2 to obtain a mixture. The mixture was heated to a temperature of 85 °C. The neutralizer and optionally the additives (emulsifiers, humectants) were added to the mixture successively, and stirred well. The temperature was lowered to 45 °C. The pH value was measured, and adjusted to 6.5 to 7.0 using a neutralizer if necessary. Then the aqueous polyurethane dispersion and optionally the preservative were added and stirred well. The pH was adjusted to 6.5 to 7.0 again with a neutralizer if necessary, to obtain the mask compositions of Examples of the present invention and of Comparative Examples.

**Table 1 Composition of mask compositions of Examples 1 to 13 of the present invention (unit: g)**

| Components | Product name | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|
| Component a) aqueous polyurethane dispersion | Baycusan® C 1004/1 | 30.0 | 30.0 | 30.0 | 30.0 | 30.0 | 30.0 | | | | | 30.0 | 19.5 | 36.6 |
| | Baycusan® C 1001/1 | | | | | | | 38.4 | | | | | | |
| | Baycusan® C 1008/1 | | | | | | | | 41.0 | | | | | |
| | Carfil 9221 | | | | | | | | | 30.8 | | | | |
| | Carfil ST11 | | | | | | | | | | 27.3 | | | |
| Component b) organic semi-synthetic water-soluble thickener | Nattosol™ 250HHR | 0.4 | 0.4 | 0.4 | 0.4 | 0.4 | 0.4 | 0.4 | 0.4 | 0.4 | 0.4 | | 0.4 | 0.4 |
| | Stardesign powder | | | | | | | | | | | 0.4 | | |
| Component c) at least one of acrylic homopolymers and crosslinked copolymers | Penmulen® TR-2 | 0.2 | 0.6 | | | 0.4 | | 0.4 | 0.4 | 0.4 | 0.4 | 0.4 | 0.4 | 0.2 |
| | Carbopol® 941 | | | 0.2 | 0.6 | | 0.4 | | | | | | | |
| additional acrylic homopolymers and crosslinked copolymers | Carbopol® 940 | 0.2 | | 0.2 | | 0.2 | | | | | | | | |
| Component d) neutralizer | Triethanol amine | 4.0 | 6.0 | 4.0 | 6.0 | 6.0 | 6.0 | 4.0 | 4.0 | 4.0 | 4.0 | 4.0 | 4.0 | 4.0 |
| Component e) additives | PEG-40 hydrogenate d castor oil | 0.5 | 0.5 | 0.5 | 0.5 | 0.5 | 0.5 | 0.5 | 0.5 | 0.5 | 0.5 | 0.5 | 0.5 | 0.5 |
| | Dipropylene glycol | 5.0 | 5.0 | 5.0 | 5.0 | 5.0 | 5.0 | 5.0 | 5.0 | 5.0 | 5.0 | 5.0 | 5.0 | 5.0 |
| | Ethanol | 5.0 | 5.0 | 5.0 | 5.0 | 5.0 | 5.0 | 5.0 | 5.0 | 5.0 | 5.0 | 5.0 | 5.0 | 5.0 |
| | Euxyl ® PE 9010 | 0.8 | 0.8 | 0.8 | 0.8 | 0.8 | 0.8 | 0.8 | 0.8 | 0.8 | 0.8 | 0.8 | 0.8 | 0.8 |
| | Water | Up to 100 | Up to 100 | Up to 100 | Up to 100 | Up to 100 | Up to 100 | Up to 100 | Up to 100 | Up to 100 | Up to 100 | Up to 100 | Up to 100 | Up to 100 |

| | | | | | | | | | | | | | | |
|---|---|---|---|---|---|---|---|---|---|---|---|---|---|---|
| Note: The above weight values refer to those including the weight of carriers. | | | | | | | | | | | | | | |

**Table 2 Composition of mask compositions of Comparative Examples 1 to 11 (unit: g)**

| Components | Product name | Comp Ex. 1 | Comp Ex. 2 | Comp Ex. 3 | Comp Ex. 4 | Comp Ex. 5 | Comp Ex. 6 | Comp Ex. 7 | Comp Ex. 8 | Comp Ex. 9 | Comp Ex. 10 | Comp Ex. 11 |
|---|---|---|---|---|---|---|---|---|---|---|---|---|
| Component a) aqueous polyurethane dispersion | Baycusan® C 1004/1 | 30.0 | 30.0 | 30.0 | 30.0 | 30.0 | 30.0 | | | | | |
| | Baycusan® C 1000/1 | | | | | | | 30.8 | | | | |
| | Baycusan® C 1001/1 | | | | | | | | 38.4 | | | |
| | Baycusan® C 1008/1 | | | | | | | | | 41.0 | | |
| | Carfil 9221 | | | | | | | | | | 30.8 | |
| | Carfil ST11 | | | | | | | | | | | 27.3 |
| Component b) organic semi-synthetic water-soluble thickener | Natrosol™ 250HHR | 0.4 | 0.4 | 0.4 | | 0.8 | | 0.4 | 0.4 | 0.4 | 0.4 | 0.4 |
| | Penmulen® TR-2 | | | | | | 0.4 | | | | | |
| | Carbopol® Ultrez 21 | 0.4 | | | | | | 0.4 | 0.4 | 0.4 | 0.4 | 0.4 |
| | Carbopol® aqua SF-1 | | 1.3 | | | | | | | | | |
| | Aculyn® 38 | | | 1.2 | | | | | | | | |
| | Carbopol® Ultrez 30 | | | | 0.5 | | | | | | | |
| Component d) neutralizer | Triethanol amine | 4.0 | 4.0 | 4.0 | 4.0 | 4.0 | 4.0 | 4.0 | 4.0 | 4.0 | 4.0 | 4.0 |
| Component e) additives | PEG-40 hydrogenat ed castor oil | 0.5 | 0.5 | 0.5 | 0.5 | 0.5 | 0.5 | 0.5 | 0.5 | 0.5 | 0.5 | 0.5 |
| | Dipropylene glycol | 5.0 | 5.0 | 5.0 | 5.0 | 5.0 | 5.0 | 5.0 | 5.0 | 5.0 | 5.0 | 5.0 |
| | Ethanol | 5.0 | 5.0 | 5.0 | 5.0 | 5.0 | 5.0 | 5.0 | 5.0 | 5.0 | 5.0 | 5.0 |
| | Euxyl ® PE 9010 | 0.8 | 0.8 | 0.8 | 0.8 | 0.8 | 0.8 | 0.8 | 0.8 | 0.8 | 0.8 | 0.8 |
| | Water | Up to 100 | Up to 100 | Up to 100 | Up to 100 | Up to 100 | Up to 100 | Up to 100 | Up to 100 | Up to 100 | Up to 100 | Up to 100 |

| | | | | | | | | | | | | |
|---|---|---|---|---|---|---|---|---|---|---|---|---|
| Note: The above weight values refer to those including the weight of carriers. | | | | | | | | | | | | |

For example, Carbopol® aqua SF-1 exists as a dispersion with a solid content of 30 wt%. The amount of Carbopol® aqua SF-1 added in Comparative Example 2 is 1.3 g. The amount of solid components of Carbopol® aqua SF-1 in Comparative Example 2 is 1.3 × 30 % = 0.4 g.

### Performance Testing

A 0.20 g sample of the mask composition was applied to the skin inside of the arm in a 4cm × 2cm area. The sample was dried to form a film, and the time from the application of the sample until it became dry was recorded as the drying time and listed in Table 4. The desired drying time was not more than 20 minutes. After wetting the mask film on the skin with water, the mask was peeled off and observed in terms of the integrity, which was listed in Table 4. The scoring principle was listed in Table 3. The desired score for the peel-off integrity score should be 5 points, which means the mask can be peeled off from the skin completely as one piece. The mask film was elongated in a direction along the original 4 cm length side until it broke. The elongation length was recorded, which was listed in Table 4. The desired stretched length is more than or equal to 23 cm. The larger the elongation length is, the better elasticity the film has. Table 3 shows the scoring principle for the peel-off integrity of mask composition samples.

**Table 3 Scoring principle for the peel-off integrity**

| Score | Peel-off integrity |
|---|---|
| 5 | Peeled off completely as one piece |
| 4 | Peel-off area at the first time >80% |
| 3 | Peel-off area at the first time >60% and ≤80% |
| 2 | Peel-off area at the first time >40% and ≤60% |
| 1 | Can't be peeled off or Peel-off area at the first time ≤40% |

Table 4 shows the performance test results of mask compositions of Examples 1 to 13. Table 5 shows the performance test results of mask compositions of Comparative Examples 1 to 11

**Table 4 Performance test results of mask compositions of Examples 1 to 13**

| | Ex.1 | Ex. 2 | Ex. 3 | Ex.4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|
| Drying time (min.) | 17.7 | 16.4 | 17.8 | 16.8 | 16.0 | 16.1 | 19.0 | 19.4 | 15.3 | 16.4 | 15.6 | 19.2 | 4.0 |
| elongation length (cm) | 24.4 | 31.5 | 23.5 | 30.6 | 30.5 | 29.6 | 31.7 | 39.2 | 30.9 | 32.7 | 29.3 | 31.0 | 2.9 |
| Score for peel-off integrity | 5 | 5 | 5 | 5 | 5 | 5 | 5 | 5 | 5 | 5 | 5 | 5 | 5 |

**Table 5 Performance test results of mask compositions of Comparative Examples 1 to 11**

| | Comp. Ex.1 | Comp. Ex. 2 | Comp. Ex. 3 | Comp. Ex. 4 | Comp. Ex. 5 | Comp. Ex. 6 | Comp. Ex. 7 | Comp. Ex. 8 | Comp. Ex. 9 | Comp. Ex. 10 | Comp. Ex. 11 |
|---|---|---|---|---|---|---|---|---|---|---|---|
| Drying time (min.) | 21.6 | 22.8 | 21.2 | 22.4 | 27.1 | 17.6 | 25.4 | 21.5 | 21.4 | 20.3 | 20.3 |
| elongation length (cm) | 22.8 | 22.6 | 22.4 | 29.5 | 19.0 | 20.1 | 9.7 | 26.9 | 27.6 | 26.4 | 29.8 |
| Score for peel-off integrity | 3 | 5 | 5 | 4 | 5 | 4 | 2 | 4 | 5 | 5 | 5 |

It can be seen from the data in Table 4 that the mask compositions of Examples 1 to 13 of the present invention had all a relatively short drying time, and the films formed by the mask compositions had all good elasticity and peel-off integrity.

It can be seen from Examples 1, 3, and 5 that the mask compositions had a relatively short drying time, and the formed films had good elasticity and peel-off integrity, when the content of the additional acrylic homopolymer and/or crosslinked copolymer contained in the mask compositions was not higher than that of component c).

The mask composition of Comparative Example 5 comprised no component c) at least one of acrylic homopolymers and crosslinked copolymers. The mask compositions of Comparative Examples 4 and 6 comprised no component b) organic semi-synthetic water-soluble thickener. It can be seen from the data in Table 5 that the mask compositions of the Comparative Examples were not able to show a combination of short drying time, good elasticity, and peel-off integrity of the formed films.

The mask composition of Comparative Example 2 comprised an acrylic copolymer, which is a polymer obtained by polymerizing two or more monomers. The polymer had a structure having at least two structural units, which were connected together by chemical bonds. The acrylic copolymer of Comparative Example 2 was different from component c) of the present invention. The mask composition of Comparative Example 2 had a long drying time and a poor elasticity of the film formed by the composition.

The mask compositions of Comparative Examples 1, 3 to 4, and 7 to 11 comprised acrylic homopolymers and crosslinked copolymers which were different from component c) of the present invention. The mask compositions of the Comparative Examples were not able to show a combination of short drying time, good elasticity, and peel-off integrity of the formed films.

### Performance of the mask compositions of Example 2 and Comparative Example 1 with halved application amount

A 0.10 g sample of the mask composition was applied to the skin inside of the arm in a 4cm × 2cm area. The sample was dried to form a film, and the time from the application of the sample until it became dry was recorded as the drying time and listed in Table 6. After wetting the mask film on the skin with water, the mask was peeled off and observed in terms of the integrity, which was listed in Table 6. The scoring principle was listed in Table 3. The mask film was stretched in a direction along the original 4 cm length side until it broke. The stretched length was recorded, which was listed in Table 6. Table 6 shows the performance test results with halved application amount of the mask compositions.

**Table 6 Performance test results with halved application amount of the mask compositions of Example 2 and Comparative Example 1**

| | Ex. 2 | Comp. Ex. 1 |
|---|---|---|
| Drying time (min.) | 10.5 | 14.2 |
| elongation length (cm) | 28 | 8.2 |
| Score for peel-off integrity | 5 | 2 |

It can be seen from Table 6 that the drying time of the mask compositions of Example 2 and Comparative Example 1 were reduced, when the amount of mask compositions applied to the skin was reduced to half. The film formed by the dry mask composition of Example 2 still had good elasticity and peel-off integrity, but the film formed by the mask composition of Comparative Example 1 had lowered elasticity and peel-off integrity, so that the basic requirements of masks could not be fulfilled. Therefore, the mask compositions of the present invention have no strict requirements on the application amount, and is more convenient for users to apply and has better user experience.

Those skilled in the art will readily understand that the present invention is not limited to the foregoing specific details. The present invention can be implemented in other specific forms without departing from the spirit or main characteristics of the present invention. The embodiments should therefore be considered as illustrative and not restrictive from any point of view. The scope of the present invention is indicated by the claims rather than by the foregoing description. Therefore, any change should be regarded as belonging to the present invention, as long as it is within the scope of equivalents of the claims.

## Claims

1. A peel-off mask composition, comprising the following components:
a) at least an aqueous polyurethane dispersion comprising a polyurethane and water;
b) at least an organic semi-synthetic water-soluble thickener;
c) at least one of acrylic homopolymers and crosslinked copolymers;
d) at least a neutralizer; and
e) optionally an additive;
wherein the amount of the polyurethane is 4 wt% to 24 wt%, the amount of the organic semi-synthetic water-soluble thickener is 0.1 wt% to 2 wt%, the sum of the amounts of the acrylic homopolymers and crosslinked copolymers is 0.1 wt% to 1 wt%, all based on the amount of the mask composition being 100 wt%;
the crosslinked copolymer is a product obtained by the crosslinking reaction of a system comprising at least one of acrylate compounds and acrylic acid compounds, at least one of sucrose allyl ether and pentaerythritol allyl ether, and an alkanol acrylate having 10 to 30 carbon atoms;
the viscosity of the acrylic homopolymer is configured as follows:
the viscosity of the aqueous acrylic homopolymer solution is less than 10000 mPa·s when the concentration of the aqueous solution is 0.2%, and
the viscosity of the aqueous acrylic homopolymer solution is less than 15000 mPa·s when the concentration of the aqueous solution is 0.5%;
the viscosity of the crosslinked copolymer is configured as follows:
the viscosity of the aqueous crosslinked copolymer solution is less than 5000 mPa·s when the concentration of the aqueous solution is 0.2%, and
the viscosity of the aqueous crosslinked copolymer solution is less than 8000 mPa·s when the concentration of the aqueous solution is 0.5%;
the viscosity being measured with the aqueous solutions adjusted to a pH value of 7.3 to 7.8 at 20 rpm using a Brookfield viscometer at 25 °C;
the aqueous solutions being prepared by dispersing the acrylic homopolymers or crosslinked copolymers in water homogeneously.

2. The mask composition according to claim 1, **characterized in that** the acrylic homopolymer has the following structure:

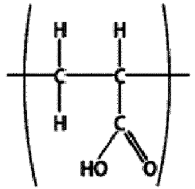

3. The mask composition according to claim 1 or 2, **characterized in that** the acrylic homopolymer is a homopolymer consisting of acrylic acid with at least one of pentaerythritol allyl ether, sucrose allyl ether and propenyl allyl ether , most preferably one or more selected from Carbopol® 941, Carbopol® 981, Carbopol® ETD 2050, Ashland™ 981-Carbomer, Synthalen®, Polygel® CB, TC-CARBOMER 381, TC-CARBOMER 341, AQUPEC® 801EG-300, AQUPEC® HV-501E, TEGO® Carbomer 141 and OptaSense G41.

4. The mask composition according to any one of claims 1 to 3, **characterized in that** the crosslinked copolymer has the following structure:

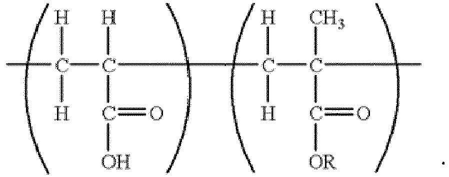

5. The mask composition according to any one of claims 1 to 4, **characterized in that** the crosslinked copolymer comprises a hydrophobic group.

6. The mask composition according to any one of claims 1 to 5, **characterized in that** the mask composition further comprises an additional acrylic homopolymer and/or crosslinked copolymer, the amount of which being less than the amount of component c) added in the composition.

7. The mask composition according to any one of claims 1 to 6, **characterized in that** the amount of component b) organic semi-synthetic water-soluble thickener is 0.2 wt% to 1 wt%, based on the amount of the mask composition being 100 wt%.

8. The mask composition according to any one of claims 1 to 7, **characterized in that** the sum of the amounts of the acrylic homopolymers and crosslinked copolymers is 0.2 wt% to 0.6 wt%, based on the amount of the mask composition being 100 wt%.

9. The mask composition according to any one of claims 1 to 8, **characterized in that** the polyurethane is obtained by the reaction of a system comprising a1) at least a water-insoluble and water-nondispersible isocyanate-functionalized polyurethane prepolymer and a2) at least an isocyanate-reactive compound.

10. The mask composition according to any one of claims 1 to 9, **characterized in that** the polyurethane comprises at least a sulfonic acid group and/or sulfonate group.

11. The mask composition according to any one of claims 1 to 10, **characterized in that** component b) organic semi-synthetic water-soluble thickener is one or more selected from modified celluloses and modified starches.

12. The mask composition according to claim 11, **characterized in that** the modified cellulose has a β-D-glucose ring with three hydroxyl groups which can undergo the nucleophilic substitution reaction, and the modified cellulose is further preferably one or more selected from cellulose ethers and derivatives of cellulose ethers, most preferably one or more selected from methyl cellulose, hydroxypropyl methyl cellulose, carboxymethyl cellulose, hydroxyethyl cellulose and hydroxypropyl cellulose.

13. The mask composition according to claim 11, **characterized in that** the modified starch is one or more selected from modified amyloses and modified amylopectins.

14. The mask composition according to any one of claims 1 to 13, **characterized in that** component b) organic semi-synthetic water-soluble thickener is hydroxyethyl cellulose.

15. The mask composition according to any one of claims 1 to 14, **characterized in that** component d) neutralizer is one or more selected from sodium hydroxide, triethanol amine, aminomethyl propanol, ammonium hydroxide, potassium hydroxide, arginine, tetrahydroxypropyl ethylenediamine, tromethamine, diisopropanol amine and triisopropanol amine.

16. A method for preparing the peel-off mask composition according to any one of claims 1 to 15, including the following steps:
i) mixing component b) organic semi-synthetic water-soluble thickener and component c) at least one of acrylic homopolymers and crosslinked copolymers to obtain a mixture; and
ii) mixing the mixture and component a) aqueous polyurethane dispersion to obtain said mask composition;
component d) and optionally component e) being each independently added in step i) or step ii).

17. Use of the peel-off mask composition according to any one of claims 1 to 15 for applying on the skin to take care of the skin.

18. A method for taking care of the skin, including:
applying the peel-off mask composition according to any one of claims 1 to 15 on the skin surface and drying to form a film;
wetting the film and peeling it off from the skin.
